# EUROPÄISCHE PATENTSCHRIFT

(11) **EP 0 669 343 B1**
(45) Veröffentlichungstag und Bekanntmachung des Hinweises auf die Patenterteilung: **15.01.2003**
(21) Anmeldenummer: 95101909.0
(22) Anmeldetag: 13.02.1995
(51) Int. Cl.: C07K 11/02, C07D 273/00, A61K 38/15, C07C 229/12, C07C 229/14, C07C 235/12, C07C 271/22

(54) **Milchsäure-haltige cyclische Depsipeptide mit 18 Ringatomen als endoparasitizide Mittel und Verfahren zu ihrer Herstellung**
Cyclic depsipeptides containing lactic acid with 18 ring atoms as endoparasiticidal agents and process for their preparation
Depsipeptides cycliques contenants de l'acide lactique avec 18 atomes cycliques comme agents endoparasiticides et procédé de leur préparation

(30) Priorität: 24.02.1994 DE 4406025
(43) Veröffentlichungstag der Anmeldung: 30.08.1995
(73) Patentinhaber: BAYER AG, 51368 Leverkusen (DE)
(72) Erfinder: Jeschke, Peter, Dr., D-51373 Leverkusen (DE); Harder, Achim, Dr. Dr., D-51109 Köln (DE); Mencke, Norbert, Dr., D-51381 Leverkusen (DE); Kleinkauf, Horst, Prof. Dr., D-12205 Berlin (DE); Zocher, Rainer, Dr., D-12169 Berlin (DE)

(56) Entgegenhaltungen:
- WO-A-93/25543
- DE-A- 2 851 629
- J.ANTIBIOTICS, Bd. 45,Nr. 8, 1992 Seiten 1273-77, R.PIEPER ET AL. 'ENNIATIN SYNTHETASES FROM DIFFERENT FUSARIA EXHIBITING DISTINCT AMINO ACID SPECIFITIES'
- J.ANTIBIOTICS, Bd. 45, 1992 Seiten 1207-15, H.TOMODA ET AL. 'NEW CYCLODEPSIPEPTIDES, ENNIATINS D,E AND F PRODUCED BY FUSARIUM sp. FO-1305'

## Beschreibung

Die vorliegende Erfindung betrifft ein neues Verfahren zur Herstellung von teilweise bekannten Milchsäure-haltigen cyclischen Depsipeptiden mit 18 Ringatomen.

Bestimmte Milchsäure-haltige cyclische Depsipeptide mit 18 Ringatomen (Enniatine) und ihre Verwendung als Endoparasitizide sind bereits Gegenstand einer vorgängigen Patentanmeldung (DE-OS 4 317 458).

Es gibt eine Reihe von chemischen und mikrobiellen Verfahren zur Herstellung von cyclischen, D-2-Hydroxy-isovaleriansäure-haltigen Depsipeptiden mit 18-Ringatomen.

(z.B. durch Synthese, vgl.: P. Quitt et al., Helv. Chimica Acta 46 (1963) S. 1715-1720; P. Quitt et al., Helv. Chimica Acta 47 (1964) S. 166-173 [Enniatin A]; Pl. A. Plattner et al., Helv. Chimica Acta 46 (1963) S. 927-935 [Enniatin B]; Yu. A. Ovchinnikov et al., Tetrahedron Lett 2 (1971) S. 159-162; R. W. Roeske et al., Biochem. Biophys. Res. Commun. 57 (1974) S. 554-561 [Beauvericin]; z.B. durch Fermentation, vgl.: R. Zocher et al., J. Antibiotics 45 (1992) S. 1273-1277 [Ermiatine A, B und C]; A. Visconti et al., J. Agric. Food Chem. 40 (1992) S. 1076-1082 [Enniatin B4]; Hiroshi Tomoda et al., J. Antibiotics 45 (1992) S. 1207-1215 [Enniatine A, A₁, B, B₁, D, E und F]).

Die Fermentation eines D-2-Hydroxy-sec.-capronsäure-haltigen Cyclohexadepsipeptids wird in einem japanischen Patent beschrieben (vgl. Synthese von MK 1688: JP-Patent 02 229 177 A2; ref. C.A. 114 (23): 227 487k).

WO 93/25543 offenbart die Verwendung von cyclischen Depsipeptiden mit 18 Ringatomen zur Bekämpfung von Endoparasiten sowie neue cyclische Depsipeptide mit 18 Ringatomen und Verfahren zu ihrer Herstellung.

Pieper et al. J. Antibiot. 45 (1992) S. 1273-1277) sowie Tomoda et al. Antibiot. 45 (1992) S. 1207-1215) offenbaren die Isolierung bestimmter Enniatin-Naturstoffe, die als D-Hydroxysäure stets D-Hydroxyisovaleriansäure enthalten

DE-OS 2 851 629 offenbart ein biologisches Peptolidpräparat, das aus Fusarium-Kulturen erhalten worden ist, sowie diese Substanz enthaltende Arzneimittel.

Über eine fermentative Darstellung Milchsäure-haltiger Cyclohexadepsipeptide (Enniatine) ist jedoch bisher nichts bekannt geworden.

Die vorliegende Erfindung betrifft ein Verfahren zur Herstellung Milchsäure-haltiger optisch aktiven, cyclischer Depsipeptide mit 18 Ringatomen der allgemeinen Formel (I) 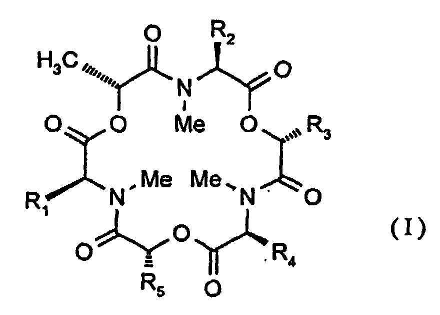 in welcher
- R¹, R² und R⁴: unabhängig voneinander für Wasserstoff, geradkettiges oder verzweigtes Alkyl mit bis zu 8 Kohlenstoffatomen, Hydroxyalkyl, Mercaptoalkyl, Alkylthioalkyl, Alkylsulfinylalkyl, Alkylsulfonylalkyl, Carboxyalkyl, Alkoxycarbonylalkyl, Carbamoylalkyl, Aminoalkyl, Alkylaminoalkyl, Dialkylaminoalkyl, Guanidinoalkyl, geradkettiges oder verzweigtes Alkenyl mit bis zu 6 Kohlenstoffatomen, cyclisches Alkyl mit bis zu 8 Kohlenstoffatomen stehen,
- R³ und R⁵: unabhängig voneinander für Wasserstoff, geradkettiges oder verzweigtes Alkyl mit bis zu 8 Kohlenstoffatomen, Hydroxyalkyl, Mercaptoalkyl, Alkylthioalkyl, Alkylsulfinylalkyl, Alkylsulfonylalkyl, geradkettiges oder verzweigtes Alkenyl mit bis zu 6 Kohlenstoffatomen, cyclisches Alkyl mit bis zu 8 Kohlenstoffatomen, sowie gegebenenfalls substituiertes, Arylalkyl oder Hetarylalkyl wobei als Substituenten genannt seien Halogen, Hydroxy, Alkoxy, Alkyl, Nitro oder Amino, stehen,
aus optisch aktiven oder racemischen Aminosäuren der Formeln (II), (III) und (IV) 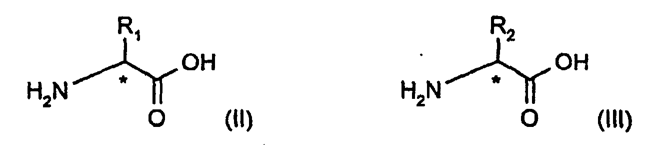 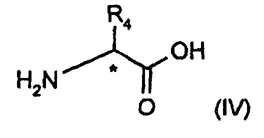 worin
R¹, R² und R⁴ die oben angegebene Bedeutung haben,
und optisch aktiven oder racemischen 2-Hydroxy-carbonsäuren der Formeln (V) und (VI) 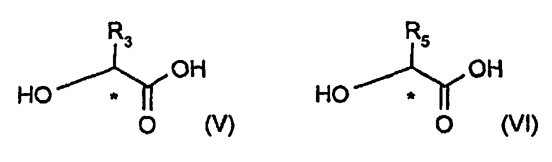 worin
R³ und R⁵ die oben angegebene Bedeutung haben, und optisch aktiver oder racemischer Milchsäure, dadurch gekennzeichnet, dass die Umsetzung in Gegenwart von Pilzstämmen der Art Fusarium in geeigneten Nährlösungen oder in Gegenwart von aus Mikroorganismen isolierten Synthetasen in einem Puffersystem erfolgt.

Die gewünschten, Milchsäure-haltigen cyclischen Depsipeptide mit 18 Ringatomen (Enniatine) werden anschließend isoliert.

Die Milchsäure-haltigen cyclischen Depsipeptide mit 18 Ringatomen (Enniatine) der allgemeinen Formel (I) eignen sich hervorragend zur Bekämpfung von Endoparasiten, besonders auf dem Gebiet der Medizin und Veterinärmedizin.

Die erfindungsgemäßen Milchsäure-haltigen cyclischen Depsipeptide mit 18 Ringatomen (Enniatine) sind durch die allgemeine Formel (I) allgemein definiert.

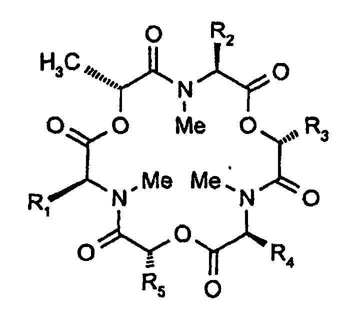

Bevorzugt sind Verbindungen der allgemeinen Formel (I)
in welcher,
- R¹, R² und R⁴: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, sec.-Pentyl, 1,2-Dimethyl-propyl, neo-Pentyl, 1-Ethyl-propyl, 1,1-Dimethyl-propyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₄-alkyl, insbesondere Hydroxymethyl, 1- und 2-Hydroxoxyethyl, Mercapto-C₁-C₄-alkyl, insbesondere Mercaptomethyl, C₁-C₄-Alkylthio-C₁-C₄-alkyl, insbesondere Methylthiomethyl, Methylthioethyl, C₁-C₄-Alkylsulfinyl-C₁-C₄-alkyl, insbesondere Methylsulfinylmethyl, Methylsulfinylethyl, C₁-C₄-Alkylsulfonyl-C₁-C₄-alkyl, insbesondere Methylsulfonylmethyl, Methylsulfonylethyl, Carboxy-C₁-C₆-alkyl, insbesondere Methoxycarbonylmethyl, Ethoxycarbonylethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₂-C₆-Alkenyl, insbesondere Vinyl, Allyl, Butenyl, Hexenyl, C₃-C₇-Cyclo-C₁-C₄-alkyl, insbesondere Cyclopropylmethyl, Cyclopentylmethyl, Cyclohexylmethyl, Cycloheptylmethyl stehen,
- R³ und R⁵: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, sec.-Pentyl, 1,2-Dimethyl-propyl, neo-Pentyl, 1-Ethylpropyl, 1,1-Dimethyl-propyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₄-alkyl, insbesondere Hydroxymethyl, 1- und 2-Hydroxoxyethyl, Mercapto-C₁-C₄-alkyl, insbesondere Mercaptomethyl, C₁-C₄-Alkylthio-C₁-C₄-alkyl, insbesondere Methylthiomethyl, Methylthioethyl, C₁-C₄-Alkylsulfinyl-C₁-C₄-alkyl, insbesondere Methylsulfinylmethyl, Methylsulfinylethyl, C₁-C₄-Alkylsulfonyl-C₁-C₄-alkyl, insbesondere Methylsulfonylmethyl, Methylsulfonylethyl, C₂-C₆-Alkenyl, insbesondere Vinyl, Allyl, Butenyl, Hexenyl, C₃-C₇-Cyclo-C₁-C₄-alkyl, insbesondere Cyclopropylmethyl, Cyclopentylmethyl, Cyclohexylmethyl, Cycloheptylmethyl, Hetaryl-C₁-C₄-alkyl, insbesondere Thien-2-yl-methyl, Thien-3-yl-methyl, Phenyl-C₁-C₄-alkyl, insbesondere Phenylmethyl das gegebenenfalls durch Reste aus der Reihe Halogen, insbesondere Fluor, Chlor, Brom oder Iod, Hydroxy, Nitro, Amino, C₁-C₄-Alkoxy, insbesondere Methoxy oder Ethoxy, C₁-C₄-Alkyl, insbesondere Methyl, substituiert sein kann, stehen.

Besonders bevorzugt sind Verbindungen der allgemeinen Formel (I),
in welcher,
- R¹, R² und R⁴: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, sec.-Pentyl, 1,2-Dimethyl-propyl, neo-Pentyl, 1-Ethyl-propyl, 1,1-Dimethyl-propyl, Hexyl, Isohexyl, sec.-Hexyl, Heptyl, Isoheptyl, sec.-Heptyl, Oktyl, Isooktyl, sec.-Oktyl, Hydroxy-C₁-C₄-alkyl, insbesondere Hydroxymethyl, 1- und 2-Hydroxoxyethyl, Mercapto-C₁-C₄-alkyl, insbesondere Mercaptomethyl, C₁-C₄-Alkylthio-C₁-C₄-alkyl, insbesondere Methylthiomethyl, Methylthioethyl, C₂-C₆-Alkenyl, insbesondere Vinyl, Allyl, Butenyl, Hexenyl, C₃-C₇-Cyclo-C₁-C₄-alkyl, insbesondere Cyclopropylmethyl, Cyclopentylmethyl, Cyclohexylmethyl, Cycloheptylmethyl, stehen,
- R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, sec.-Pentyl, 1,2-Dimethyl-propyl, neo-Pentyl, 1-Ethyl-propyl, 1,1-Dimethyl-propyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₄-alkyl, insbesondere Hydroxymethyl, 1- und 2-Hydroxoxyethyl, C₁-C₄-Alkylthio-C₁-C₄-alkyl, insbesondere Methylthiomethyl, C₂-C₆-Alkenyl, insbesondere Vinyl, Allyl, Butenyl, Hexenyl, C₃-C₇-Cyclo-C₁-C₄-alkyl, insbesondere Cyclopropylmethyl, Cyclopentylmethyl, Cyclohexylmethyl, Cycloheptylmethyl, stehen.

Ganz besonders bevorzugt sind Verbindungen der Formel ( I ),
in welcher
- R¹, R² und R⁴: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, sec.-Pentyl, 1,2-Dimethyl-propyl, neo-Pentyl, 1-Ethylpropyl, 1,1-Dimethyl-propyl, Hexyl, Isohexyl, sec.-Hexyl, Heptyl, Isoheptyl, sec.-Heptyl, Oktyl, Isooktyl, sec.-Oktyl, C₂-C₆-Alkenyl, insbesondere Vinyl, Allyl, Butenyl, C₃-C₇-Cyclo-C₁-C₄-alkyl, insbesondere Cyclohexylmethyl, stehen,
- R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, sec.-Pentyl, 1,2-Dimethyl-propyl, neo-Pentyl, 1-Ethyl-propyl, 1,1-Dimethyl-propyl, Hexyl, Isohexyl, sec.-Hexyl, Heptyl, Isoheptyl, sec.-Heptyl, tert.-Heptyl, Oktyl, Isooktyl, sec.-Oktyl, C₂-C₆-Alkenyl, insbesondere Vinyl, Allyl, Butenyl, Hexenyl, C₃-C₇-Cyclo-C₁-C₄-alkyl, insbesondere Cyclohexylmethyl, stehen.

Im einzelnen seien folgende optisch aktiven Verbindungen der allgemeinen Formel (I) genannt:
Cyclo(-N-methyl-L-alanyl-D-lactyl-N-methyl-L-isoleucyl-D-lactyl-N-methyl-Lvalyl-D-lactyl-),
Cyclo(-N-methyl-L-alanyl-D-lactyl-N-methyl-L-isoleucyl-D-lactyl-N-methyl-Lnorvalyl-D-lactyl-),
Cyclo(-N-methyl-L-alanyl-D-lactyl-N-methyl-L-isoleucyl-D-lactyl-N-methyl-Lleucyl-D-lactyl-),
Cyclo(-N-methyl-L-alanyl-D-lactyl-N-methyl-L-isoleucyl-D-lactyl-N-methyl-Lnorleucyl-D-lactyl-),
Cyclo(-N-methyl-L-valyl-D-lactyl-N-methyl-L-valyl-D-2-hydroxy-isovaleryl-N-methyl-L-valyl-D-lactyl-),
Cyclo(-N-methyl-L-valyl-D-2-hydroxy-isovaleryl-N-methyl-L-valyl-D-2-hydroxyisovaleryl-N-methyl-L-valyl-D-lactyl-),
Cyclo(-N-methyl-L-alanyl-D-lactyl-N-methyl-L-alloisoleucyl-D-lactyl-N-methyl-Lalloisoleucyl-D-lactyl-),
Cyclo(-N-methyl-L-alanyl-D-lactyl-N-methyl-L-alloisoleucyl-D-lactyl-N-methyl-Lalanyl-D-lactyl-),
Cyclo(-N-methyl-L-alanyl-D-lactyl-N-methyl-L-isoleucyl-D-lactyl-N-methyl-L-2-amino-butyryl-D-lactyl-),
Cyclo(-N-methyl-L-alanyl-D-lactyl-N-methyl-L-alloisoleucyl-D-lactyl-N-methyl-L-2-amino-butyryl-D-lactyl-).

Die Verbindungen der allgemeinen Formel (I) sind teilweise bekannt (vgl. z.B.: V. Z. Pletnev et al. Bioorg. Khim. 1 (2) (1975) S. 160-165; ref. C.A. 83 (13): 114 872e; DE-OS 4 317 458 und können auch nach den dort angegebenen chemisch synthetischen Verfahren erhalten werden.

Setzt man bei dem erfindungsgemäßen Verfahren zur Herstellung der Milchsäure-haltigen cyclischen Depsipeptide (Enniatine) (I) als Verbindungen der Formeln (II) bis (IV) L-Valin (R¹, R² und R⁴: -Isopropyl) und als Verbindungen der Formeln (V) und (VI) D-Milchsäure (R³ und R⁵: -Methyl) ein, so läßt sich das Verfahren z.B. durch folgendes Reaktionsschema wiedergeben: 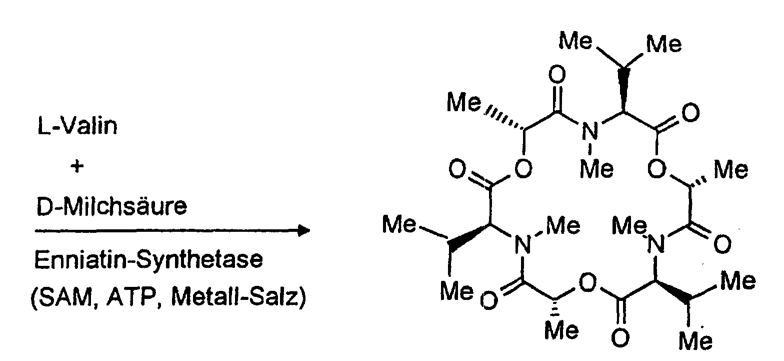
SAM: S-Adenosyl-L-methionin
ATP: Adenosintriphosphat
Metall-Salz: z.B. Erdalkalimetall-Salz (Mg²⁺-Salz) oder Mn²⁺-Salz

Die zur Durchführung des erfindungsgemäßen Verfahrens als Ausgangsstoffe benötigten Aminosäuren sind durch die Formeln (II) bis (IV) allgemein definiert. In diesen Formeln stehen R¹, R² und R⁴ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsstoffe verwendeten natürlichen oder synthetischen Aminosäuren können, falls chiral, in der D- oder L-Form vorliegen. Bevorzugt sind jedoch L-konfigurierte alpha-Aminosäuren.

Beispielsweise seien genannt:
Aad, Abu, jAbu, ABz, 2ABz, εAca, Ach, Acp, Adpd, Ahb, Aib, βAib, Ala, βAla, ΔAla,
Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hlle, hLeu, hLys, hMet, hPhe, hPro, hSer, hThr, hTrp, hTyr, HyI, Hyp, 3Hyp, Ile, Ise, Iva, Kyn, Lant, Lcn, Leu, Lsg, Lys, ßLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Nal, Tbg, Npg, Chg, Thia, (vgl. z.B. Houben-Weyl, Methoden der Organischen Chemie, Band XV/1 und 2, Stuttgart, 1974).

Die zur Durchführung des erfindungsgemäßen Verfahrens als Ausgangsstoffe benötigten 2-Hydroxy-carbonsäuren sind durch die Formeln (V) bis (VI) allgemein definiert.
In diesen Formeln stehen R³ und R⁵ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsstoffe verwendeten 2-Hydroxy-carbonsäuren können, falls chiral, in der D- oder L-Form vorliegen. Bevorzugt sind jedoch die D-konfigurierten 2-Hydroxycarbonsäuren.

Beispielsweise seien genannt:
Hyac, Hyba, Hydd, Hyde, Hyic, Hyiv, Hymb, Hypp, Hypr (Lac), Hytd, Hyud, Hyva, (vgl. z.B. Houben-Weyl, Methoden der Organischen Chemie, Band XV/1 und 2, Stuttgart, 1974).

Geeignete Fusarium Stämme zur Durchführung des erfindungsgemäßen Verfahrens sind die nachfolgenden Fusarium Stämme:

| Fusarium Stamm | | isoliert aus |
|---|---|---|
| Fusarium acuminatum | | |
| BBA 62 148 | | Schmalblättrige Lupine |
| | | |
| Fusarium arthrosporoides | | |
| BBA 64 134 | | Straußgras (Samen) |
| | | |
| Fusarium avenaceum | | |
| BBA 64 338 | | Wintergerste (Samen) |
| BBA 62 163 | | Gemüsekohl |
| | | |
| Fusarium compactum | | |
| BBA 65 671 | | Baumwolle |
| | | |
| Fusarium crookwellense | | |
| BBA 64 297 | | Weizen (Stengelgrund) |
| | | |
| Fusarium ensiforme | | |
| BBA 64 683 | | Süßkartoffel |
| | | |
| Fusarium equiseti | | |
| BBA 64 814 | | Roggen |
| | | |
| Fusarium inflexum | | |
| BBA 63 203 | | Saubohne |
| | | |
| Fusarium gibbosum | | |
| | | |
| Fusarium lateritium | | |
| BBA 65 090 | | Weizen (Stengelgrund) |
| | | |
| Fusarium meresmoides | | |
| BBA 64 329 | | Roggen (Stengelgrund) |
| | | |
| Fusarium moniliforme | | |
| | | |
| Fusarium oxysporum | | |
| BBA 62 057 | f. pisi | Erbse |
| BBA 62 060 | f. lycopersici | Tomate |
| BBA 62 334 | f. lupini | Weiße Lupine |
| BBA 64 952 | f. batatas | Süßkartoffel |
| | | |
| Fusarium proliferatum | | |
| BBA 63 625 | | Drachenbaum |
| | | |
| Fusarium redolens | | |
| BBA 62 390 | | Gartennelke |
| | | |
| Fusarium sambucinum | | |
| BBA 63 933 | | Weizen |
| BBA 62 397 | | Kartoffel |
| NRRL-13 500 | | Kartoffel |
| NRRL-13 503 | | Kartoffel |
| R-583 | | Knöterich |
| R-5390 | | Katoffel |
| R-7570 | | Boden |
| R-5455 | | Getreide |
| R-6380 | | Kartoffel |
| R-7843 | | Nelke |
| R-5690 | | Boden |
| R-2633 | | Kartoffel |
| R-6354 | | Getreide |
| | | |
| Fusarium scirpi | | |
| ETH 1536 | | Weideland-Boden |
| | | |
| Fusarium semitectum | | |
| | | |
| Fusarium solani | | |
| BBA 64 953 | | Süßkartoffel |
| BBA 62 420 | f. pisi | Erbse |
| | | |
| Fusarium subglutinans | | |
| | | |
| Fusarium tricinctum | | Wiesenklee |
| BBA 62 446 | | |
| | | |
| Fusarium udum | | Cajanus inidians |
| BBA 62 451 | | |

Insbesondere seien die am 31.01.1994 bei der Deutschen Sammlung für Mikroorganismen (DSM) in Braunschweig gemäß Budapester Vertrag Mintolyte Fusarium Stämme DSM 8938 und DSM 8939 genannt.

Das Verfahren kann auch mit aus Mikroorganismen isolieren Synthetasen durchgeführt werden. Die dafür benötigten Enniatin-Synthetasen können aus den weiter oben genannten Fusarium Stämmen nach literaturbekannten Verfahren isoliert werden (vgl. z.B.: R. Pieper, H. Kleinkauf, R. Zocher, J. Antibiot. 45 (1992) S. 1273-1277).

Die Fermentation der Pilzstämme der Art Fusarium erfolgt nach an sich bekannten Methoden in Gegenwart geeigneter Nährlösungen. Diese Nährlösungen enthalten die für das Wachstum der Pilze notwendigen Salze sowie Kohlenstoff- und Stickstoffquellen.

Als geeignete anorganische Salze zur Durchführung des erfindungsgemäßen Verfahrens kommen alle Alkali-, Erdalkali- und Metall-Salze mit Elementen der II. bis VIII. Nebengruppe des Periodensystems in Frage.

Beispielhaft seien dafür erwähnt die Acetate, Chloride, Bromide, Iodide, Fluoride, Nitrate, Nitrite, Phosphate, Hydrogenphosphate, Dihydrogenphosphate, Phosphite, Hydrogenphosphite, Sulfate, Hydrogensulfate, Sulfite, Hydrogensulfite, Carbonate, Hydrogencarbonate des Lithiums, Natriums, Kaliums, Caesiums, Magnesiums, Calciums, Bariums, Zinks, Cadmiums, Scandiums, Titans, Zirkoniums, Vanadiums, Niobs, Chroms, Molybdäns, Mangans, Eisen, Cobalts oder Nickels.

Vorzugsweise verwendet man die Acetate, Halogenide, Phoshate, Hydrogenphosphate, Dihydrogenphosphate und Nitrate der Alkalimetalle, insbesondere Natrium und Kalium, die Sulfate der Erdalkalimetalle, insbesondere Magnesium, und Metalle der II, VII und VIII. Nebengruppe des Periodensystems, beispielsweise Zink, Mangan und Eisen.

Als Kohlenstoff-Quelle zur Durchführung des erfindungsgemäßen Verfahrens kommen Kohlenhydrate und kohlenhydrathaltige Produkte in Frage.

Beispielhaft seien dafür erwähnt die Monosaccharide wie Pentosen, insbesondere Ribose, die Hexosen, insbesondere Glucose und Fructose, die Oligosaccharide wie Disaccharide, insbesondere Saccharose, Maltose und Lactose, die Trisaccharide, insbesondere Raffinose, sowie die Tetra-, Penta- und Hexasaccharide.

Vorzugsweise verwendet man Monosaccharide wie beispielsweise Hexosen, insbesondere Glucose, und Oligosaccharide wie beispielsweise Disaccharide, insbesondere Saccharose.

Als Stickstoff-Quelle zur Durchführung des erfindungsgemäßen Verfahrens kommen Aminosäuren und stickstoffhaltige Salze in Frage.

Beispielhaft seien dafür die weiter oben genannten natürlichen und synthetischen Aminosäuren erwähnt oder stickstoffhaltige Salze wie Ammoniumnitrat, Ammoniumnitrit oder die Nitrate und Nitrite der weiter oben genannten Metalle.

Vorzugsweise verwendet man die weiter oben genannten natürlichen Aminosäuren sowie stickstoffhaltige Salze wie Ammoniumnitrat.

Die für das fermentative Verfahren verwendeten Fusarienstämme werden zunächst nach an sich bekannten Methoden in einem Medium, bestehend z.B. aus Melasse/Cornsteep-Liquor, angezüchtet. Nach erfolgter Kultivation werden die entstandenen Sporen über Sporenfilter isoliert. Zur Darstellung der Vorkultur wird ein Fusarium definiertes Medium (FDM), bestehend aus einer Kohlenstoff-Quelle und anorganischen Salzen, mit ca. 10⁹ Sporen angeimpft und erneut fermentiert. Nach wenigen Tagen kann die FDM-Hauptkultur durch Überimpfen von je 1 ml Vorkultur hergestellt und analog fermentiert werden.

Die eigentliche Fermentation wird dann in Gegenwart von Verbindungen der Formeln (II) bis (IV) oder (V) und (VI) und in Gegenwart von optisch aktiver oder racemischer Milchsäure durchgeführt.

Die Fermentationsdauer beträgt 1 bis 30 Tage. Die Fermentation erfolgt bei Temperaturen zwischen +5°C und +40°C, bevorzugt zwischen +15°C und +35°C, besonders bevorzugt zwischen +25°C und +30°C. Es wird steril und unter Normaldruck gearbeitet.

Zur Durchführung werden die Verbindungen der Formeln (V) und (VI) im allgemeinen in einer Konzentration von 5 mM bis 50 mM, vorzugsweise 5 mM bis 15 mM eingesetzt.

Nach vollendeter Fermentation wird das Mycel der Fusarienkultur abgesaugt, homogenisiert, mehrmals mit einem organischen Lösungsmittel extrahiert und anschließend filtriert. Das anfallende Kulturfiltrat wird in üblicher Weise extrahiert, getrocknet und im Vakuum eingeengt.

Die anfallenden Rohenniatine lassen sich in üblicher Weise durch Säulenchromatographie oder durch Craig-Verteilung reinigen. Welches das optimale Verfahren ist, muß von Fall zu Fall entschieden werden. (vgl. auch die Herstellungsbeispiele).

Wird das erfindungsgemäße Verfahren in Gegenwart von isolierten Synthetasen durchgeführt wird in einem wäßrigen Puffersystem in Gegenwart von Metallsalzen, S-Adenosyl-L-methionin (SAM) und Adenosintriphosphat (ATP) gearbeitet.

Als Metallsalze seien genannt: Acetate, Chloride, Bromide, Iodide, Fluoride, Nitrate, Phosphate, Hydrogenphosphate, Phosphite, Hydrogenphosphite, Sulfate, Hydrogensulfate, Sulfite, Hydrogensulfite, Carbonate und Hydrogencarbonate des Lithiums, Natriums, Kaliums, Caesiums, Magnesiums, Calciums oder Bariums.

Vorzugsweise verwendet man Erdalkalimetall-Salze, wie beispielsweise Magnesiumchlorid, -sulfat oder -acetat.

Das erfindungsgemäße Verfahren wird in einer wässrigen Pufferlösung durchgeführt.

Beispielhaft seien dafür kommerziell erhältliche Pufferlösungen erwähnt, z.B. für den pH-Wert 1,0, insbesondere Glycin-Salzsäure, für den pH-Wert 2,0 bis 4,0, insbesondere Citrat-Salzsäure, für den pH-Wert 5,0 bis 6,0, insbesondere Citrat-Natronlauge, für den pH-Wert 7,0, insbesondere Phosphat, für den pH-Wert 8,0, insbesondere Borat-Salzsäure, und für den pH-Wert 9,0 bis 10,0, insbesondere Borsäure/Kaliumchlorid-Natronlauge.

Vorzugsweise arbeitet man im "physiologischen Bereich", d.h. bei einem pH-Wert von 6,0 bis 9,0 und verwendet dafür bevorzugt eine Phosphatpufferlösung, insbesondere Kaliumhydrogenphosphat/Dinatriumhydrogenphosphat oder Kaliumhydrogenphosphat/Dikaliumhydrogenphosphat.

Zur Durchführung werden im allgemeinen 2 mM bis 8 mM, vorzugsweise 3 mM bis 5 mM an Verbindungen der Formeln (II) bis (VI), optisch aktiver oder racemischer Milchsäure und S-Adenosyl-L-methionin (SAM), 3 mM bis 9 mM, vorzugsweise 4 mM bis 6 mM Adenosintriphosphat (ATP), und 2 mM bis 25 mM, vorzugsweise 5 mM bis 15 mM an Erdalkalimetall-Salz, 10 mM bis 100 mM, vorzugsweise 40 mM bis 60 mM Puffer mit 100 µg bis 1000 µg, vorzugsweise 200 µg bis 600 µg isolierter Enniatin-Synthethase in-vitro eingesetzt.

Die Reaktionsdauer der enzymatischen in-vitro-Synthese beträgt 2 Minuten bis 24 Stunden. Die enzymatische in-vitro-Synthese erfolgt in einem Temperaturbereich von 0°C bis +50°C, vorzugsweise bei +10°C bis +35°C, besonders bevorzugt zwischen +20°C und +30°C.

Sie verläuft in einem pH-Bereich von 6,5 bis 8,5, vorzugsweise bei 7,0 bis 8,0, wobei der pH-Wert durch Zugabe eines Puffers während der gesamten Reaktion bei 7,3 konstant gehalten wird.

Vorzugsweise arbeitet man unter sterilen Reaktionsbedingungen und bei Normaldruck.

Die enzymatische in-vitro-Synthese kann durch Verdünnen mit Wasser gestoppt werden.

Zur Aufarbeitung wird . die wässrige Phase mehrmals mit einem organischen Lösungsmittel extrahiert, getrocknet und im Vakuum eingeengt.

Die anfallenden Rohenniatine lassen sich in üblicher Weise durch Säulenchromatographie oder durch Craig-Verteilung reinigen. Welches das optimale Verfahren ist, muß auch hier von Fall zu Fall entschieden werden. (vgl. auch die Herstellungsbeispiele).

### Herstellungsbeispiele

### Beispiel 1:

### Herstellung von Cyclo(-N-methyl-L-valyl-D-lactyl-N-methyl-L-valyl-D-lactyl-N-methyl-L-valyl-D-lactyl-)

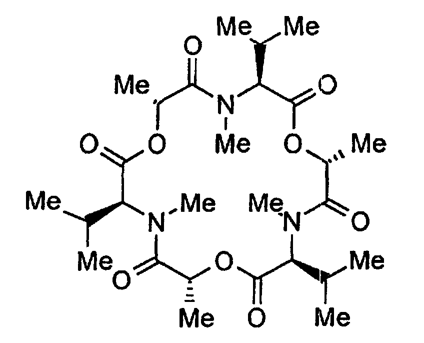

### In-vivo-Incorporation von D-Milchsäure

Zu einer 2 Tage alten Hauptkultur von Fusarium scirpi wird D-Milchsäure in einer Konzentration von 10 mM steril zugegeben und die Fermentation noch 3 Tage fortgesetzt. Danach wird das Mycel der Fusarienkultur in einer Nutsche abgesaugt und das Filtrat dreimal mit Essigsäureethylester extrahiert. Das Mycelium wird zweimal mit Aceton im Mörser homogenisiert und anschließend abgesaugt. Das Kulturfiltrat wird dreimal mit je 100 ml Essigsäureethylester ausgeschüttelt und die gesammelten organischen Phasen zusammen mit dem Acetonextrakt zur Trockene eingedampft.

Alternativ kann auch die gesamte Fusarienkultur über Nacht mit einem ca. zweifachen Volumen Essigsäureethylester extrahiert werden.

Zur säulenchromatographischen Anreicherung des Enniatins wird das in wenig Chloroform gelöste Rohenniatin auf eine Al₂O₃-Säule (30 x 2 cm) gegeben und stufenweise eluiert.

### Enzymatische in-vitro-Synthese

300 - 500 µg gereinigte Enniatin-Synthetase in 50 mM Phosphatpuffer (pH 7,3) werden in einem Gesamtvolumen von 1,5 ml in Gegenwart von 4 mM L-Valin, 4 mM D-Milchsäure, 4 mM S-Adenosyl-L-methionin (SAM), 5 mM Adenosintriphosphat (ATP) und 10 mM MgCl₂ 10 Minuten bei 28 °C inkubiert.

Nach Zugabe von 2 ml Wasser wird mehrmals mit 2 ml Portionen Essigsäureethylester extrahiert. Die organische Phase wird nach dem Trocknen mit Natriumsulfat im Vakuum eingeengt.

Die Aufreinigung des nach a) oder b) erhaltenen Produktes erfolgt mittels präparativer HPLC (RP 18 / 75-80% Methanol).

¹H-NMR (400 MHz, CDCl₃, δ): 0,83; 1,03 (d, 18H, 3 x -CH(CH₃)₂); 1,45 (d, 9H, 3 x -O-CH-CH₃); 2,27 (m, 3H, 3 x -CH(CH₃)₂); 3.06 (s, 9H, 3 x -N-CH₃); 4,43 (d, 3H, 3 x -N-CH-CO-); 5,62 (q, 3H, 3 x -O-CH-CO-) ppm
¹³C-NMR (100 MHz, CDCl₃, δ): 16,5 (3 x -CH₃, D-Lac); 18,5; 20,1 (6 x -CH₃, MeVal); 27,8 (3 x -CH(CH₃)₂, L-MeVal); 32,9 (3 x -N-CH₃, L-MeVal); 63,1 (3 x -N-CH-CO-, L-MeVal); 66,3 (3 x -O-CH-CO-, D-Lac); 169,2 (3 x -C=O, Amid); 169,8 (3 x -C=O Ester) ppm
EI-MS m/z (%): 555 (M⁺, 32); 482 (20); 353 (1); 268 (34); 168 (100); 86 (53)

### Beispiel 2

### Herstellung von Cyclo(-N-methyl-L-isoleucyl-D-lactyl-N-methyl-L-isoleucyl-Dlactyl-N-methyl-L-isoleucyl-D-2-hydroxy-isovaleryl-)

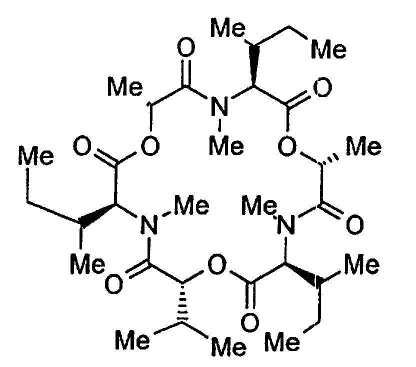

### In-vivo-Incorporation von D-Milchsäure

Zu einer 2 Tage alten Hauptkultur von Fusarium sambucinum wird D-Milchsäure in einer Konzentration von 10 mM steril zugegeben und die Fermentation noch 3 Tage fortgesetzt. Danach wird das Mycel der Fusarienkultur in einer Nutsche abgesaugt und das Filtrat dreimal mit Essigsäureethylester extrahiert. Das Mycelium wird zweimal mit Aceton im Mörser homogenisiert und anschließend abgesaugt. Das Kulturfiltrat wird dreimal mit je 100 ml Essigsäureethylester ausgeschüttelt und die gesammelten organischen Phasen zusammen mit dem Acetonextrakt zur Trockne eingedampft.

Die Anreicherung und Aufreinigung des Enniatins erfolgt wie bei Beispiel 1.

¹³C-NMR (100 MHz, CDCl₃, δ): 18,4; 18,0 (2 x -CH₃, D-HyIv); 16,8; 15,8 (2 x-CH₃, D-Lac); 16,7; 15,3; 11,4; 10,6; 10,3; 10,0 (6 x -CH₃, L-Melle); 25,0; 24,9; 24,4 (3 x -CH₂-, L-Melle); 34,7; 34,1; 32,3 (3 x -CH(CH₃)-, L-Melle); 35,6; 31,6; 31,2 (3 x -N-CH₃, L-Melle); 29,9 (-CH(CH₃)₂, D-HyIv); 74,0; 67,5; 66,1 (3 x -O-CH-CO-, D-HyIv); 65,1; 60,5; 59,5; (3 x -N-CH-CO-, L-Melle); 169,2; 169,1; 169,0 (3 x -C=O, Amid); 170,6; 170,1; 169,8 (3 x -C=O, Ester) ppm
EI-MS m/z (%): 625 (M⁺, 23); 552 (14); 409 (5); 296 (20); 182 (43); 100 (100)

Analog können die in der nachstehenden Tabelle 1 aufgeführten Verbindungen der allgemeinen Formel (I) als LDLDLD-Stereoisomere hergestellt werden.

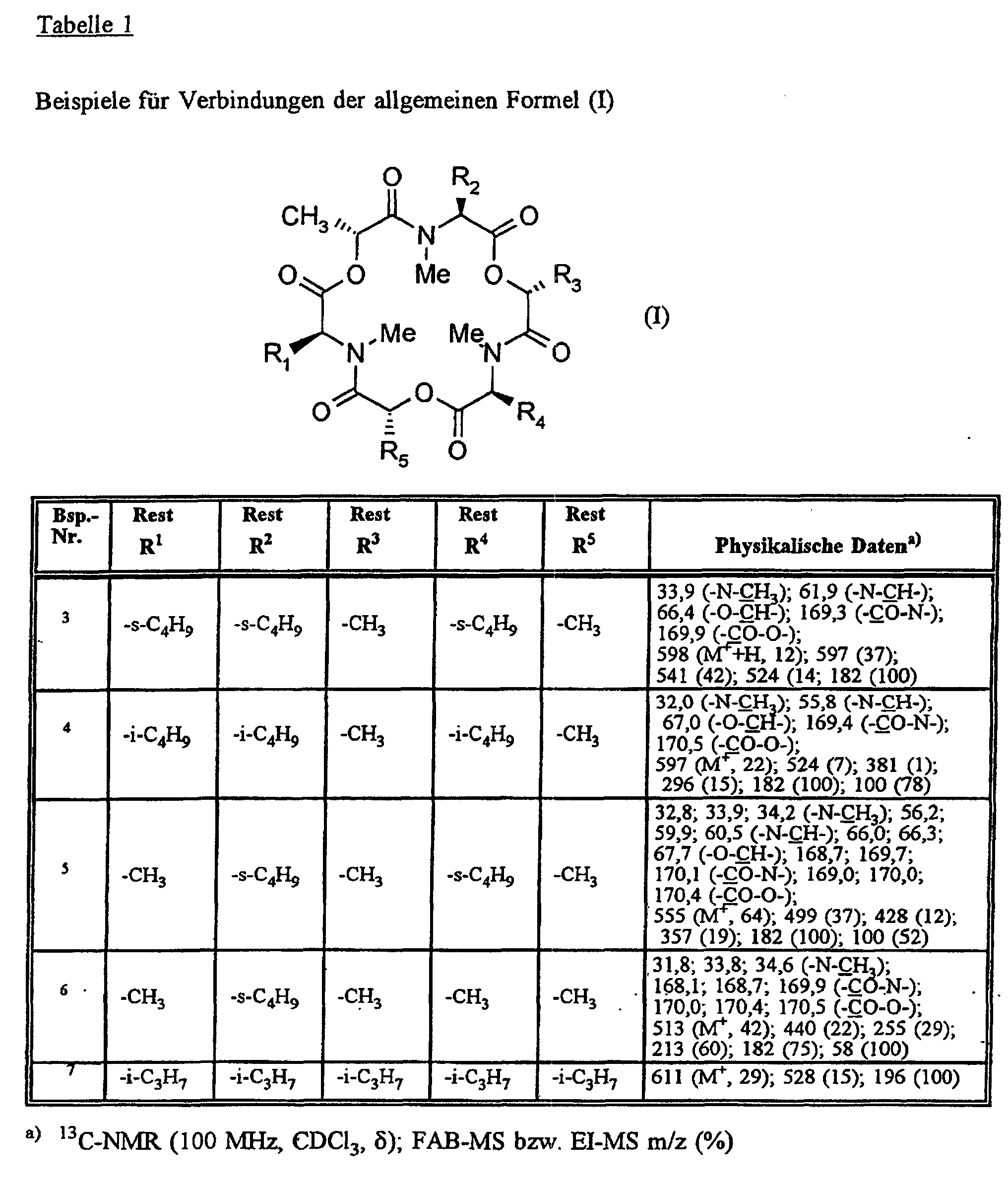

### Kultivation der Sporen, Vorkulturen und Hauptkulturen

Der betreffende Fusarium-Stamm wird in einem Medium bestehend aus Melasse/Cornsteep-Liquor (30 g bzw. 10 g/l) angezüchtet.

Die Kultivation erfolgt in 500 ml Erlenmeyerkolben (100 ml Medium) bei 100 rpm (26 bis 28°C). Nach 4 bis 5 Tagen werden die entstandenen Sporen über ein Sporenfilter isoliert. Diese können über Wochen bei 4°C aufbewahrt werden.

Zur Herstellung einer Vorkultur wird ein Kolben mit 200 ml FDM (75,0 g Saccharose, 12,75 g NaNO₃, 15,0 g NaCl, 7,5 g MgSO₄ · 7 H₂O, 4,0 g KH₂PO₄ · 7 H₂O, 10 g ZnSO₄ pro Liter) mit 10⁹ Sporen angeimpft und wie oben fermentiert.

Nach 2 bis 3 Tagen werden FDM-Hauptkulturen durch überimpfen von je 1 ml Vorkultur hergestellt und wie oben fermentiert.

### Vorbereitung der gezielten in-vivo-Enniatin-Synthese

2 bis 3 Tage alte Hauptkulturen werden zunächst auf ihren Enniatintiter untersucht um zu gewährleisten, daß die Zellen syntheseaktiv sind. Dazu werden 3 bis 5 ml Kultur steril entnommen und mehrmals mt je 2 ml Essigsäureethylester extrahiert. Nach Eindampfen der organischen Phase wird das Enniatin direkt durch HPLC (RP 18,80 % Methanol) bestimmt.

Zu den betreffenden Hauptkulturen wird die entsprechende Präkursor Hydroxyoder Aminosäure bis zu einer Endkonzentration von 10 mM steril zugegeben und die Fermentation, wie im Beispiel 1 erläutert, fortgesetzt (Gesamtdauer ca. 1 Woche).

## Patentansprüche

1. Verfahren zur Herstellung von Milchsäure-haltigen optisch aktiven, cyclischen Depsipeptiden mit 18 Ringatomen (Enniatine) der allgemeinen Formel (I) 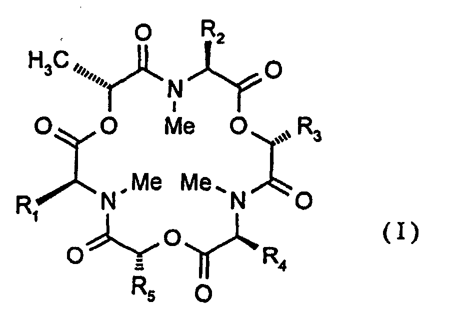 in welcher
R¹, R² und R⁴ unabhängig voneinander für Wasserstoff, geradkettiges oder verzweigtes Alkyl mit bis zu 8 Kohlenstoffatomen, Hydroxyalkyl, Mercaptoalkyl, Alkylthioalkyl, Alkylsulfinylalkyl, Alkylsulfonylalkyl, Carboxyalkyl, Alkoxycarbonylalkyl, Carbamoylalkyl, Aminoalkyl, Alkylaminoalkyl, Dialkylaminoalkyl, Guanidinoalkyl, geradkettiges oder verzweigtes Alkenyl mit bis zu 6 Kohlenstoffatomen, cyclisches Alkyl mit bis zu 8 Kohlenstoffatomen stehen,
R³ und R⁵ unabhängig voneinander für Wasserstoff, geradkettiges oder verzweigtes Alkyl mit bis zu 8 Kohlenstoffatomen, Hydroxyalkyl, Mercaptoalkyl, Alkylthioalkyl, Alkylsulfinylalkyl, Alkylsulfonylalkyl, geradkettiges oder verzweigtes Alkenyl mit bis zu 6 Kohlenstoffatomen, cyclisches Alkyl mit bis zu 8 Kohlenstoffatomen, sowie gegebenenfalls substituiertes, Arylalkyl oder Hetarylalkyl wobei als Substituenten genannt seien Halogen, Hydroxy, Alkoxy, Alkyl, Nitro oder Amino, stehen,
aus optisch aktiven oder racemischen Aminosäuren der Formeln (II), (III) und (IV) 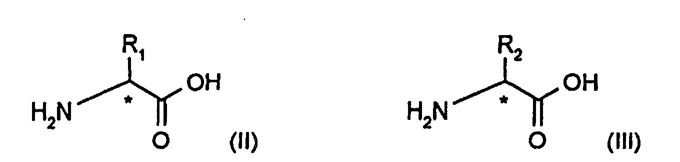 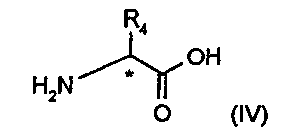 worin
R¹, R² und R⁴ die oben angegebene Bedeutung haben,
und optisch aktiven oder racemischen 2-Hydroxy-carbonsäuren der Formeln (V) und (VI) 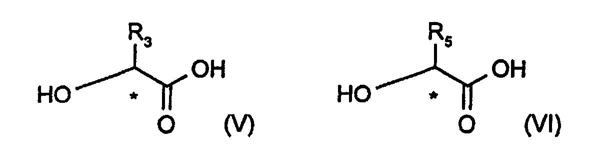 worin
R³ und R⁵ die oben angegebene Bedeutung haben,
und optisch aktiver oder racemischer Milchsäure, **dadurch gekennzeichnet, daß** die Umsetzung in Gegenwart von Pilzstämmen der Art Fusarium in geeigneten Nährlösungen oder in Gegenwart von aus Mikroorganismen isolierten Synthetasen in einem Puffersystem erfolgt.

2. Verfahren gemäß Anspruch 1 zur Herstellung von Verbindungen der allgemeinen Formel (I)
in welcher
R¹, R² und R⁴ unabhängig voneinander für Wasserstoff, geradkettiges oder verzweigtes C₁-C₈-Alkyl, Hydroxy-C₁-C₄-alkyl, Mercapto-C₁-C₄-alkyl, C₁-C₄-Alkylthio-C₁-C₄-alkyl, C₁-C₄-Alkylsulfinyl-C₁-C₄-alkyl, C₁-C₄-Alkylsulfonyl-C₁-C₄-alkyl, Carboxy-C₁-C₆-alkyl, Carbamoyl-C₁-C₆-alkyl, Amino-C₁-C₆-alkyl, C₁-C₄-Alkylamino-C₁-C₆-alkyl, C₁-C₄-Dialkylamino-C₁-C₆-alkyl, Guanido-C₁-C₆-alkyl, C₂-C₆-Alkenyl, C₃-C₇-Cyclo-C₁-C₄-alkyl stehen,
R³ und R⁵ unabhängig voneinander für Wasserstoff, geradkettiges oder verzweigtes C₁-C₈-Alkyl, Hydroxy-C₁-C₄-alkyl, Mercapto-C₁-C₄-alkyl, C₁-C₄-Alkylthio-C₁-C₄-alkyl, C₁-C₄-Alkylsulfinyl-C₁-C₄-alkyl, C₁-C₄-Alkylsulfonyl-C₁-C₄-alkyl, C₂-C₆-Alkenyl, C₃-C₇-Cyclo-C₁-C₄-alkyl, Hetaryl-C₁-C₄-alkyl, Phenyl-C₁-C₄-alkyl, das gegebenenfalls durch Reste aus der Reihe Halogen, insbesondere Fluor, Chlor, Brom oder Iod, Hydroxy, Nitro, Amino, C₁-C₄-Alkoxy, insbesondere Methoxy oder Ethoxy, C₁-C₄-Alkyl, insbesondere Methyl, substituiert sein kann, stehen.

## Claims

1. Process for the preparation of lactic-acid-containing, optically active, cyclic depsipeptides having 18 ring atoms (enniatins) of the general formula (I) 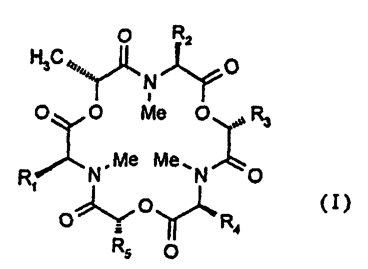 in which
R¹, R² and R⁴ independently of one another represent hydrogen, straight-chain or branched alkyl having up to 8 carbon atoms, hydroxyalkyl, mercaptoalkyl, alkylthioalkyl, alkylsulphinylalkyl, alkylsulphonylalkyl, carboxyalkyl, alkoxycarbonylalkyl, carbamoylalkyl, aminoalkyl, alkylaminoalkyl, dialkylaminoalkyl, guanidinoalkyl, straight-chain or branched alkenyl having up to 6 carbon atoms, cyclic alkyl having up to 8 carbon atoms,
R³ and R⁵ independently of one another represent hydrogen, straight-chain or branched alkyl having up to 8 carbon atoms, hydroxyalkyl, mercaptoalkyl, alkylthioalkyl, alkylsulphinylalkyl, alkylsulphonylalkyl, straight-chain or branched alkenyl having up to 6 carbon atoms, cyclic alkyl having up to 8 carbon atoms, and optionally substituted arylalkyl or hetarylalkyl, substituents which may be mentioned being halogen, hydroxyl, alkoxy, alkyl, nitro or amino,
from optically active or racemic amino acids of the formulae (II), (III) and (IV) 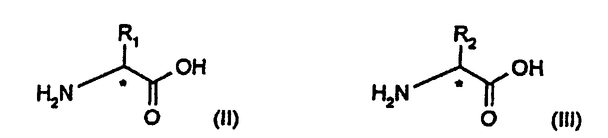 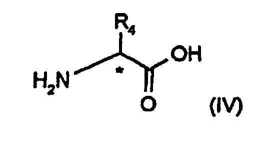 in which
R¹, R² and R⁴ have the abovementioned meaning,
and optically active or racemic 2-hydroxycarboxylic acids of the formulae (V) and (VI) 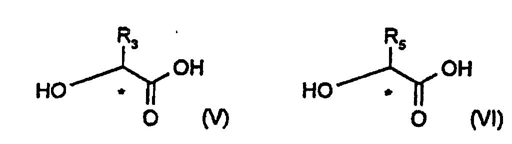 in which
R³ and R⁵ have the abovementioned meaning,
and optically active or racemic lactic acid, **characterized in that** the reaction is carried out in the presence of fungal strains of the species Fusarium in suitable nutrient solutions or in a buffer system in the presence of synthetases isolated from microorganisms.

2. Process according to Claim 1 for the preparation of compounds of the general formula (I)
in which
R¹, R² and R⁴ independently of one another represent hydrogen, straight-chain or branched C₁-C₈-alkyl, hydroxy-C₁-C₄-alkyl, mercapto-C₁-C₄-alkyl, C₁-C₄-alkylthio-C₁-C₄-alkyl, C₁-C₄-alkylsulphinyl-C₁-C₄-alkyl, C₁-C₄-alkylsulphonyl-C₁-C₄-alkyl, carboxy-C₁-C₆-alkyl, carbamoyl-C₁-C₆-alkyl, amino-C₁-C₆-alkyl, C₁-C₄-alkylamino-C₁-C₆-alkyl, C₁-C₄-dialkylamino-C₁-C₆-alkyl, guanido-C₁-C₆-alkyl, C₂-C₆-alkenyl, C₃-C₇-cyclo-C₁-C₄-alkyl,
R³ and R⁵, independently of one another, represent hydrogen, straight-chain or branched C₁-C₈-alkyl, hydroxy-C₁-C₄-alkyl, mercapto-C₁-C₄-alkyl, C₁-C₄-alkylthio-C₁-C₄-alkyl, C₁-C₄-alkylsulphinyl-C₁-C₄-alkyl, C₁-C₄-alkylsulphonyl-C₁-C₄-alkyl, C₂-C₆-alkenyl, C₃-C₇-cyclo-C₁-C₄-alkyl, hetaryl-C₁-C₄-alkyl, phenyl-C₁-C₄-alkyl which can optionally be substituted by radicals from the series consisting of halogen, in particular fluorine, chlorine, bromine or iodine, hydroxyl, nitro, amino, C₁-C₄-alkoxy, in particular methoxy or ethoxy, C₁-C₄-alkyl, in particular methyl.

## Revendications

1. Procédé de production de depsipeptides cycliques optiquement actifs contenant de l'acide lactique, à noyau de 18 atomes (enniatines) de formule générale (I) 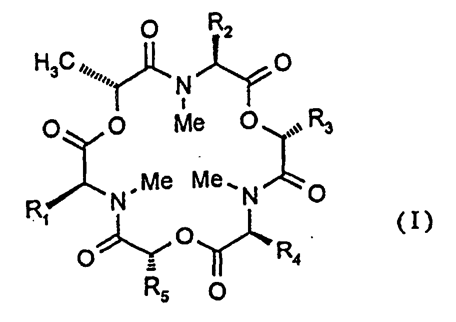 dans laquelle
R¹, R² et R⁴ représentent, indépendamment les uns des autres, l'hydrogène, un groupe, linéaire ou ramifié, alkyle ayant jusqu'à 8 atomes de carbone, hydroxyalkyle, mercapto-alkyle, alkylthio-alkyle, alkylsulfinylalkyle, alkylsulfonylalkyle, carboxyalkyle, alkoxycarbonylalkyle, carbamoylalkyle, amino-alkyle, alkylamino-alkyle, dialkylamino-alkyle, guanidinoalkyle, un groupe alcényle linéaire ou ramifié ayant jusqu'à 6 atomes de carbone, un groupe alkyle cyclique ayant jusqu'à 8 atomes de carbone,
R³ et R⁵ représentent, indépendamment l'un de l'autre, l'hydrogène, un groupe, linéaire ou ramifié, alkyle ayant jusqu'à 8 atomes de carbone, hydroxyalkyle, mercapto-alkyle, alkylthio-alkyle, alkylsulfinylalkyle, alkylsulfonylalkyle, un groupe alcényle linéaire ou ramifié ayant jusqu'à 6 atomes de carbone, un groupe alkyle cyclique ayant jusqu'à 8 atomes de carbone, ainsi qu'un groupe arylalkyle ou hétarylalkyle éventuellement substitué, les substituants pouvant être mentionnés étant un radical halogéno, hydroxy, alkoxy, alkyle, nitro ou amino,
à partir d'amino-acides optiquement actifs ou racémiques de formules (II), (III) et (IV) 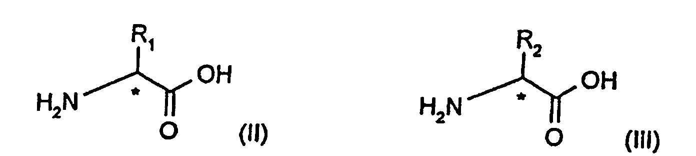 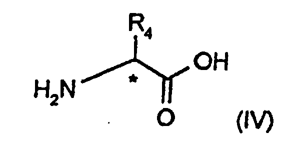 dans lesquelles
R¹, R² et R⁴ ont la définition indiquée ci-dessus, et d'acides 2-hydroxycarboxyliques optiquement actifs ou racémique de formules (V) et (VI) 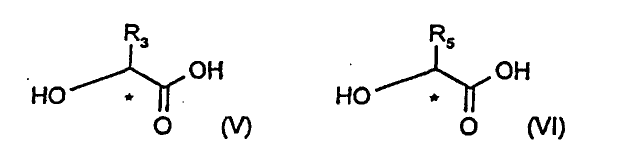 dans lesquelles
R³ et R⁵ ont la définition indiquée ci-dessus,
et d'acide lactique optiquement actif ou racémique, **caractérisé en ce que** la réaction est conduite en présence de souches de champignons du genre Fusarium dans des solutions nutritives appropriées ou en présence de synthétases isolées de micro-organismes dans un système tampon.

2. Procédé suivant la revendication 1 pour la production de composés de formule générale (I)
dans laquelle
R¹, R² et R⁴ représentent, indépendamment les uns des autres, l'hydrogène, un groupe linéaire ou ramifié alkyle en C₁ à C₈, hydroxyalkyle en C₁ à C₄, mercapto-alkyle en C₁ à C₄, (alkylthio en C₁ à C₄)-(alkyle en C₁ à C₄), (alkylsulfinyle en C₁ à C₄)-(alkyle en C₁ à C₄), (alkylsulfonyle en C₁ à C₄)-(alkyle en C₁ à C₄), carboxy-(alkyle en C₁ à C₄), carbamoyl-(alkyle en C₁ à C₆), amino-alkyle en C₁ à C₆, (alkylamino en C₁ à C₄)-(alkyle en C₁ à C₆), di-(alkyle en C₁ à C₄)amino-(alkyle en C₁ à C₆), guanidino-(alkyle en C₁ à C₆), alcényle en C₂ à C₆, (cyclo-alkyle en C₃ à C₇)-(alkyle en C₁ à C₄),
R³ et R⁵ représentent, indépendamment l'un de l'autre, l'hydrogène, un groupe, linéaire ou ramifié, alkyle en C₁ à C₈, hydroxyalkyle en C₁ à C₄, mercapto-alkyle en C₁ à C₄, (alkylthio en C₁ à C₄)-(alkyle en C₁ à C₄), (alkylsulfinyle en C₁ à C₄)-(alkyle en C₁ à C₄), (alkylsulfonyle en C₁ à C₄)-(alkyle en C₁ à C₄), alcényle en C₂ à C₆, (cycloalkyle en C₃ à C₇)-(alkyle en C₁ à C₄), hétaryl-(alkyle en C₁ à C₄), phényl-(alkyle en C₁ à C₄), qui peut éventuellement être substitué par des restes de la série halogène, en particulier fluor, chlore, brome ou iode, hydroxy, nitro, amino, alkoxy en C₁ à C₄, en particulier méthoxy ou éthoxy, alkyle en C₁ à C₄, en particulier méthyle.
